# EUROPEAN PATENT SPECIFICATION

(11) **EP 1 682 672 B1**
(45) Date of publication and mention of the grant of the patent: **14.10.2009**
(21) Application number: 03772448.1
(22) Date of filing: 14.11.2003
(51) Int. Cl.: C12Q 1/68

(54) **METHOD OF DETECTING PREDISPOSITION TO HIGH ALTITUDE PULMONARY EDEMA**
VERFAHREN ZUM NACHWEIS EINER PRÄDISPOSITION FÜR HÖHENBEDINGTES LUNGENÖDEM
PROCEDE DE DETECTION D'UNE PREDISPOSITION A L'OEDEME PULMONAIRE DE HAUTE MONTAGNE

(43) Date of publication of application: 26.07.2006
(73) Proprietor: Council of Scientific and Industrial Research, New Delhi 110 001 (IN)
(72) Inventor: PASHA, Abdul Qadar Mohammad, 110 007 New Delhi (IN); AHSAN, Aarif, 110 007 New Delhi (IN)
(74) Representative: Srinivasan, Ravi Chandran
(86) International application number: PCT/IB2003/005158
(87) International publication number: WO 2005/047540

(56) References cited:
- DATABASE EMBL [Online] EBI; Retrieved on 04.06.2004, "Alignment display for SEQ ID NO:1" retrieved from EBI Database accession no. AC131306 XP002283507
- DATABASE GENBANK [Online] Partial sequence, 19 February 1904 (1904-02-19) "H.sapiens, chromosome 17, genomic contig" retrieved from NCBI Database accession no. NT_010799 XP002283508
- BASNYAT B ET AL: "High-altitude illness" LANCET THE, LANCET LIMITED. LONDON, GB, vol. 361, no. 9373, 7 June 2003 (2003-06-07), pages 1967-1974, XP004429770 ISSN: 0140-6736
- DROMA YUNDEN ET AL: "Positive association of the endothelial nitric oxide synthase gene polymorphisms with high-altitude pulmonary edema" CIRCULATION, vol. 106, no. 7, 13 August 2002 (2002-08-13), pages 826-830, XP002283504 ISSN: 0009-7322 cited in the application
- WEISS JOHANNA ET AL: "Lack of evidence for association of high altitude pulmonary edema and polymorphisms of the NO pathway." HIGH ALTITUDE MEDICINE & BIOLOGY. UNITED STATES 2003 FALL, vol. 4, no. 3, October 2003 (2003-10), pages 355-366, XP001181946 ISSN: 1527-0297
- XU WEIMING ET AL: "Molecular cloning and structural organization of the human inducible nitric oxide synthase gene (NOS2)" BIOCHEMICAL AND BIOPHYSICAL RESEARCH COMMUNICATIONS, vol. 219, no. 3, 1996, pages 784-788, XP002283505 ISSN: 0006-291X -& DATABASE GENBANK [Online] H.sapiens NOS2 gene, exons 8 and 9, 19 August 1996 (1996-08-19) retrieved from NCBI Database accession no. X85766 XP002283548
- CHARTRAIN NICOLE A ET AL: "Molecular cloning, structure, and chromosomal localization of the human inducible nitric oxide synthase gene" JOURNAL OF BIOLOGICAL CHEMISTRY, vol. 269, no. 9, 1994, pages 6765-6772, XP002283506 ISSN: 0021-9258
- DATABASE SNP [Online] SNP in iNOS gene at pos. 845034 of NT_010799, 11 May 2003 (2003-05-11) retrieved from NCBI Database accession no. RS2297520 XP002283509

## Description

### TECHNICAL FIELD

The present invention relates to a method for the detection of predisposition to high altitude pulmonary edema (HAPE). It particularly relates with the allelic variants of iNOS (inducible nitric oxide synthase) gene, which has been found to be related with the prevalence of HAPE.

### BACKGROUND AND PRIOR ART

High altitude pulmonary edema (HAPE) is a form of noncardiogenic pulmonary edema that develops in approximately 10% of randomly selected mountaineers within 24h after rapid ascent to altitude above 4,000 m. A similar phenomenon is observed in the lowlander inductees to a height above 3000 m for various business reasons. An even higher incidence rate of about 60% has been demonstrated in subjects who are susceptible to HAPE as documented by previous occurrence of the disease (Houston CS et al 1960, Bartsch P et al 1997, 1990). HAPE can be effectively prevented by prophylactic use of vasodilators or slow ascent. Nevertheless, it remains the most common cause of death related to high altitude exposure during trekking or mountaineering (Hackett PH et al 1990). The morbidity rate in Himalayan mountaineers was estimated to be 50% if immediate treatment with supplemental oxygen or rapid descent is impossible (Lobenhoffer HP et al 1982). Observed differences in clinical presentations and severity of the disease between racial and ethnic groups together with familial clustering favor a significant hereditary predisposition to the disease. ,

Although knowledge of the factors influencing the development of HAPE is still incomplete, there is experimental evidence that an exaggerated hypoxic pulmonary vasoconstriction (HPV) plays an important role (Scherrer U et al 1996). An excessive rise in pulmonary artery pressure has been demonstrated by invasive and noninvasive measurements at high altitude in individuals with HAPE. The uneven vasoconstriction in the capillaries sometimes results in "capillary leakage" followed by edema formation (Bartsch P et al 1991). Human subjects who are susceptible to the disease demonstrate an increased pulmonary vascular response even during a brief exposure of high altitude. The underlying pathophysiological mechanism for this exaggerated HPV is still unknown. There is, however, evidence that the endogenous vasodilator nitric oxide (NO) modulates vascular reactivity (Palmer RMJ et al 1987). Regulation of vascular tone by NO is attributed to the intermediates of cGMP pathway (Bellamy TC et al 2002).

The following studies emphasize the involvement of NO in HAPE:

NO exerts its effect mainly via improvement of ventilation/perfusion ratio and lowering of alveolar to arterial oxygen tension difference by increasing arterial oxygen saturation (Scherrer U et al 1996). However, in the healthy volunteers, administration of the NO synthesis antagonist N^{G}-monomethyl-L-arginine (L-NMMA) during hypoxia increases pulmonary artery pressure and vascular resistance which is similar to that observed in HAPE. Due to this NO has been used as an inhalation therapy for the treatment of HAPE in the affected individuals (Anand IS et al 1998).

Phosphodiesterase 5 is the key enzyme responsible for cGMP hydrolysis in the lungs. The inhibitors of Phosphodiesterase 5 have been found to inhibit hypoxia induced pulmonary hypertension (Goldstein I et al 1998). Hypoxia decreases exhaled NO in mountaineers susceptible to HAPE indicating decreased NO production in such cases (Busch et al 2001). Thus defective NO synthesizing machinery imparting lower NO level may be envisaged to be responsible for the pathogenesis of HAPE. NO is synthesized by three isozymes nNOS (neuronal nitric oxide synthase, NOS1), iNOS (inducible nitric oxide synthase, NOS2) and eNOS (endothelial nitric oxide synthase, NOS3) (Michel T et al 1997). NOS1 and NOS3 are constitutively expressed while NOS2 is expressed upon induction. Among these the best candidate which is supposed to be defective in HAPE is eNOS (endothelial nitric oxide synthase) while induction of iNOS (inducible nitric oxide synthase) seems to be inevitable for the immediate recovery of the total NO reserve (Xia Y et al 1998). Moreover, robust cell signaling mechanisms generally favor the recruitment of inducible genes for immediate early physiological responses. It can be speculated that a defect in iNOS which doesnot permit its activation may not recover the reduced NO level in individuals exposed to hypoxia resulting in HAPE.

The defect in iNOS may occur at genetic level in HAPE patients. In numerous cases, the expression of the genes has been found to get altered by the polymorphisms in the gene sequence (Qadar Pasha MA et al 2001). Hence, it is always possible that polymorphism in iNOS gene may alter its expression and associates with the disease.

### Current status of the treatment of HAPE:

1. NO therapy: NO is being used as an inhalation therapy for the treatment of HAPE. It exerts its effect mainly via improvement of ventilation/perfusion ratio and lowering of alveolar to arterial oxygen tension difference by increasing arterial oxygen saturation. NO induced improvement in arterial oxygenation in subjects with HAPE was accompanied by a shift in blood flow in the lung away from edematous segments and toward nonedematous segments results in evening/homogeneity of the vasoconstriction throughout the capillaries (Scherrer U et al 1996, Anand IS et al 1998).
2. Rapid descent: Rapid descent of HAPE patients not only prevents the worsening but even improves the pathogenesis of the disease (Hackett PH et al 2001).
3. Portable Air Chambers (PACs): PACs in the form of small cylinders filled with oxygen is often used as inhalation therapy for HAPE (Hackett PH et al 2001).
4. Genetic predisposition: The only study in this context suggests that genetic variation in endothelial nitric oxide synthase gene (eNOS) and angiotensin converting enzyme gene (ACE) may predispose individuals to HAPE (Droma Y et al 2002). The results are as follows:

| | Controls | Patients |
|---|---|---|
| Glu298Asp (eNOS) | 9.8% | 25.6% |
| B/A (eNOS) | 6.9% | 32.2% |
| I/D (ACE) | 4% | 22% |

### Limitations of the available therapies for HAPE:

1. HAPE patients do not found to have homogenous response to NO inhalation. Moreover, concentration of required NO varies with the severity of the disease. Sometimes inadequate inhalation results in hypotension or even septic shock to the patients.
2. Immediate descent of the HAPE patients often remains impossible due to severe weather and rugged terrain (Anand IS et al 1998, Hackett PH et al 2001).
3. Carriage of PACs sometimes appears to be not feasible due to overloading problem. Improved conditions of the disease are often temporary as removal of chambers renders the patient worse (Hackett PH et al 2001).
4. The reported polymorphisms associated with HAPE are not specific but have also been shown to be associated with the disorders like diabetes, coronary artery disease, hypertension and myocardial infarction where elevated blood pressure is observed (Monti LD et al 2003, Via M et al 2003). The allelic frequency difference mentioned appears to be the same with other diseases. Hence the possibility of allelic contribution to the disease may be due to other related pathophysiologies like hypertension, which involves the exacerbations of HAPE. Moreover, the study does not include HA natives (high landers), a population residing blissfully in the same environment where the disease occurs.

Novelty of the invention is in providing a novel method for the detection of predisposition to HAPE.

Still another novelty is for providing a novel marker region in iNOS gene.

Still another novelty is to demonstrate association of the allelic variants of iNOS gene with HAPE.

Another novelty is to provide novel primers for amplification of the SNP.

### OBJECTS OF THE INVENTION:

Main object of the present invention is to provide a method for the detection of predisposition to HAPE, which obviates the limitations listed above.

Another object is to provide novel primers for amplification of the SNP.

Another object is to perform association analysis for the allelic variants between low landers and HAPE patients so that the relation with the disease could be scored.

### SUMMARY OF THE INVENTION:

The present invention relates to the method of detection of predisposition to HAPE. It particularly relates with the allelic variants of iNOS gene, which has been related to the prevalence of HAPE. Defective Nitric Oxide (NO) synthesizing machinery imparting lower NO level has been envisaged to be responsible for the pathogenesis of HAPE. iNOS gene has been shown to be responsible for NO production as the inhibitors of NO production increased the severity of HAPE. Present invention provides a method for detection of predisposition to HAPE as an allelic variant of iNOS gene in the disclosed marker region was shown to be negatively associated with the prevalence of HAPE in a population.

### BRIEF DESCRIPTION OF ACCOMPANYING FIGURES/DRAWINGS

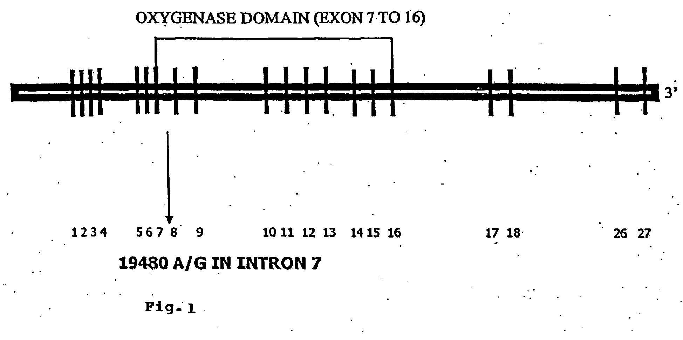

Figure 1 Schematic representation of the gene of inducible Nitric Oxide Synthase (iNOS) localization: 17 cenq^{11.2}. The vertical bars showing the exonic regions (From Gene bank Nucelotide Sequence ID No. NT_010799).

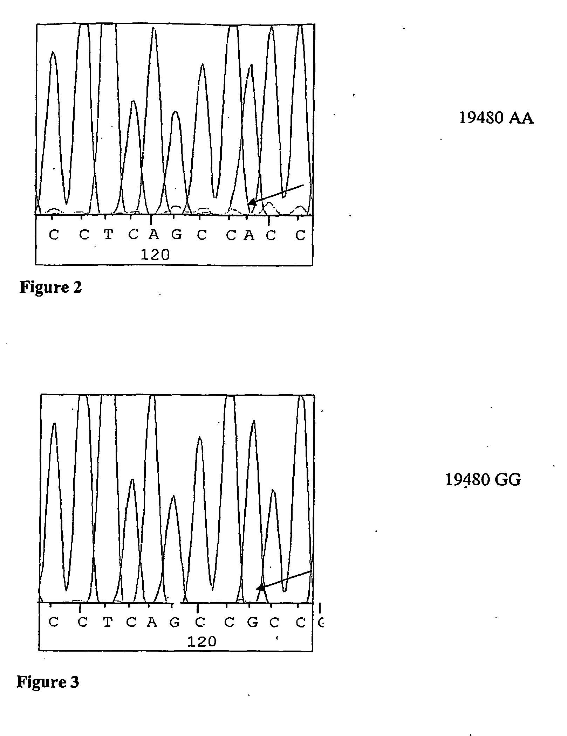

Figure 2 shows sequence file of the individual with AA homozygote.
Figure 3 shows sequence file of the individual with GG homozygote.

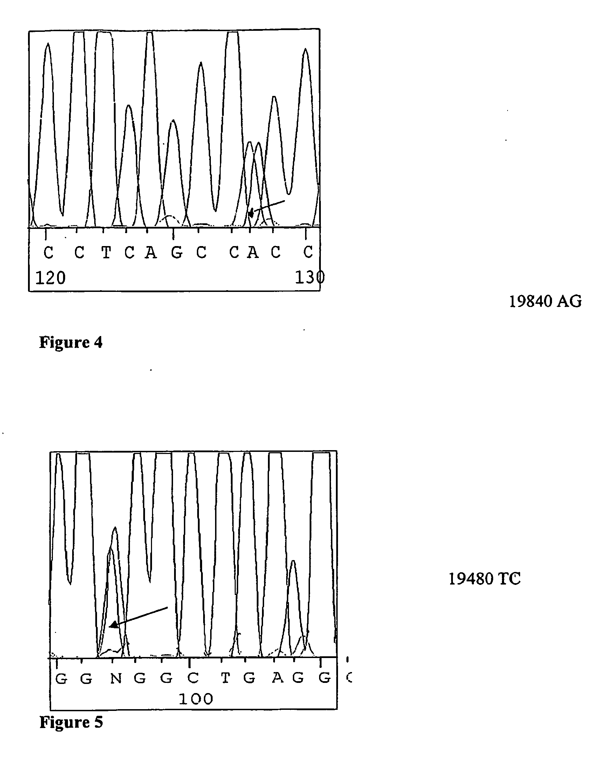

Figure 4 shows sequence file of the individual with AG heterozygote.
Figure 5 shows sequence file of the individual with TC heterozygote.

### DETAILED DESCRIPTION OF THE INVENTION

The present invention relates to the method of detection of predisposition to HAPE. It particularly relates with the allelic variant of iNOS gene, which has been found to be related to the prevalence of HAPE.

### I. Identification of the marker region on the iNOS gene:

Taking in consideration the important functions of NO at high altitude, iNOS, the inducible nitric oxide synthase gene was selected as the candidate gene for the study.

### II. Selection of the study subjects:

Clinical severity of HAPE was assessed by Lake Louise acute mountain sickness (AMS) scoring system. Briefly, the patients were assessed for the presence of five symptoms: headache, gastrointestinal upset, fatigue, weakness, or both, dizziness, lightheadedness, or both, and difficulty in sleeping. Change in mental status, ataxia and peripheral edema were also assessed. Each of these symptoms were rated between 0 and 3. A score of 0 indicated no symptoms; 1, mild symptoms; 2, moderate symptoms; and 3, severe symptoms. HAPE score is the sum of all 8 symptoms and patients were characterized by HAPE score>6 (Anand IS et al 1998). Lowlanders (LLs) were subjects who even after induction to high altitudes atleast thrice never found to have any of the above mentioned symptoms. High altitude (HA) natives were the permanent residents of HA from ancient times.

### III. Extraction of genomic DNA from leukocytes:

Genomic DNA was extracted from blood using salting out method. Lysis of red blood cells in presence of high salt was followed by treatment with Nucleus lysis buffer (NLB). Proteins were precipitated and extraction of DNA was obtained in ethanol (Miller SA.et al 1988).

### IV. Identification of the allelic variants of the iNOS gene:

As a first step to the present invention, the applicants carried out the PCR amplification of marker region of the iNOS gene using self designed oligonucleotide primers. The primers were designed in accordance with the human iNOS gene sequence (Gene Bank Accession Number NT_010799). The sequencing of the purified PCR product revealed a single nucleotide polymorphism. Disclosed herein is a sequence for the allelic variant of human iNOS gene comprising the following single nucleotide polymorphism compared with the human iNOS gene sequence in the database.

For example, the nucleotide sequence of the allelic variant of human iNOS gene (SEQ ID NO: 1) having the polymorphic site listed in Table 1 may be-

5'CAGCGGAGTGATGGCAAGCACGACTTCCGGGTGTGGAATGCTCAGCT
CATCCGCTATGCTGGCTACCAGATGCCAGATGGCAGCATCAGAGGGGA
CCCTGCCAACGTGGAATTCACTCAGGTACCCGGCCCAGCCTCAGCC
A*/GCCGGCCATTGGGGCGGGGAGCCCCGTGGTGAGCGAGTGACAGAGT
GGAGCCCAGAGGAGACACGCAGCCCGGGCTTACAGACTCACAGGGCCC
GTCTTGTTCCCCAGCTGTGCATC3'

In the above sequence the SNP* is shown in bold.

**Table 1**

| Site of change | Base change | Mutation type |
|---|---|---|
| 19480 | A/G | Transition |

### V. Association Analysis with the disease

Analysis of the SNP in 42 HA natives, 39 HAPE controls and 18 HAPE patients revealed three genotypes, namely AA, AG and GG. The distribution of alleles is summarized in Table 2.

**Table 2**

| Study subjects | A | G |
|---|---|---|
| HAPE controls (n=39) | 0.35 | 0.65 |
| HAPE patients (n=18) | 0.58 | 0.42 |
| HA natives (n=42) | 0.18 | 0.82 |

The frequency of the G allele was found to be in the order of HA natives>HAPE controls>HAPE subjects. The biostatistical analysis showed a significant association of G allele with HA adaptation and A allele with the disease as mentioned in Table 3. Herein the odds ratio (OR) and 95% confidence of interval was used as a measure of the strength of the association between genotypic combination and the disease. P value of <0.05 was considered statistically significant.

**Table 3**

| Association type | χ² value | p value | Odds ratio | 95% CI | Relative risk |
|---|---|---|---|---|---|
| HAPE patients & HAPE controls | 10.63 | 0.001 | 2.56 | 1.45-4.54 | 1.66 (1.21-2.27) |
| HAPE patients & HA natives | 33.96 | <0.001 | 6.29 | 3.30-12.01 | 3.22 (2.05-5.06) |
| HAPE controls & HA natives | 7.42 | 0.006 | - | - | - |

Nitric oxide synthase for its reaction to synthesize nitric oxide, requires oxygen which acts as a cofactor in the reaction. Oxygen binds to the oxygenase domain in iNOS and contributes to the synthesis of NO. In hypoxic condition scarcity of oxygen may lead to lower NO production, however any modification in the oxygenase domain, which modify the activity of the enzyme in such a way that it requires no oxygen or less oxygen may contribute to normal NO production. NO improves oxygenation of hemoglobin and normal NO production may involve the mechanisms acting in acclimatization, hence any alteration in oxygenase domain may be favorable for the production of NO. In the present investigation the SNP is present near to the oxygenase domain of NOS2 gene which spans exon 7 to exon 16. It is quite possible that the SNP found is in linkage disequilibrium to a nearby SNP, which is contributing to the final impact on NO production by NOS2 gene.

### VI. Diagnostic kits

The invention further provides diagnostic kit comprising the oligonucleotides as described in SEQ ID 2 and 3. Often, the kits contain one or more pairs of oligonucleotides hybridizing to different forms of a polymorphism. In some kits, the oligonucleotides are provided immobilized to a substrate. For example, the same substrate can comprise allele-specific oligonucleotide probes for detecting at least the polymorphism shown in Table1. Optional additional components of the kit include, for example, restriction enzymes, reverse transcriptase or polymerase, the substrate nucleoside triphosphates, means used to label (for example, an avidin enzyme conjugate and enzyme substrate and chromogen if the label is biotin), and the appropriate buffers for reverse transcription, PCR, or hybridization reactions. Usually, the kit also contains instructions for carrying out the methods.

### VII. Nucleic acid vectors

Variant genes can be expressed in an expression vector in which a variant gene is operably linked to a native or other promoter. Usually, the promoter is eukaryotic promoter for expression in a mammalian cell. The transcription regulation sequences typically include a heterologous promoter and optionally an enhancer, which is recognized by the host. The selection of an appropriate promoter, for example trp, lac, phage promoters, glycolytic enzyme promoters and tRNA promoters, depends on the host selected. Commercially available expression vectors can also be used. Suitable host cells include bacteria such as E.coli, yeast, filamentous fungi, insect cells, mammalian cells, typically immortalized, e.g., mouse, CHO, human and monkey cell lines and derivatives thereof. Preferred host cells are able to process the variant gene product to produce an appropriate mature polypeptide.

We describe transgenic non-human animals capable of expressing an exogenous variant gene and/or having achieved by operably linking the gene to a promoter and optionally an enhancer, and microinjecting the construct into a zygote. Inactivation of endogenous variant genes can be achieved by forming a transgene in which a cloned variant gene is inactivated by insertion of a positive selection marker. The transgene is then introduced in to an embryonic stem cell, where it undergoes homologous recombination with an endogenous variant gene. Mice and other rodents are preferred animals. Such animals provide useful drug screening systems.

Accordingly, the main embodiment of the present invention relates to a method for detecting predisposition to high altitude pulmonary edema (HAPE) in a subject, said method comprising identifying a polymorphism in an inducible nitric oxide synthase gene of said subject, wherein said polymorphism is at position 142 of SEQ ID NO: 1, wherein predisposition to HAPE is indicated by the presence of the nucleotide A at said position. We further describe a method comprising the steps of:
(a) selecting study subjects by monitoring high altitude pulmonary edema associated symptoms,
(b) extracting genomic DNA from leukocytes by conventional methods from the study subjects,
(c) amplifying the human iNOS gene sequence of SEQ ID No.1 by designing and synthesizing Forward and Reverse oligonucleotide primers of SEQ ID No. 2 and SEQ ID No. 3, respectively,
(d) identifying computationally novel Single Nucleotide Polymorphism (SNP) by comparing with the already existing sequence of human iNOS gene,
(e) screening the high altitude native population (HA natives), low lander natives (HAZE controls) and low lander HAPE patients for the novel single nucleotide polymorphism, using above said primers of SEQ ID No.2 (Forward Primer) and SEQ ID 3 (Reverse Primer),
(f) computing the frequencies of AA, AG and GG genotypes in the populations of step (e) for establishing the association of the genotypes with high altitude pulmonary edema, and
(g) predicting and statistically analyzing the differences in the distribution of the allelic variants (AA, AG and GG genotypes) in the populations wherein GG genotype at 19480 position are at low risk to high altitude pulmonary edema and AA genotype at 19480 position are at high risk of the disease.

Another embodiment of the present invention relates to the oligonucleotide primers capable for amplification of human iNOS gene selected from group comprising of
(a) 5' CAG CGG AGT GAT GGC AAG CAC GAC 3' (SEQ ID No. 2), which is a forward primer, and
(b) 5' GAT GCA CAG CTG GGG AAC AAG ACG 3' (SEQ ID No. 3), which is a reverse primer.

A diagnostic kit for the detection of SNP genotypes having predisposition to high altitude pulmonary edema (HAPE) said kit comprising a pair of primers:
(a) 5' CAG CGG AGT GAT GGC AAG CAC GAC 3' (SEQ ID No.2), which is a forward primer
(b) 5' GAT GCA CAG CTG GGG AAC AAG ACG 3' (SEQ ID No.3), which is a reverse primer

One more embodiment of the present invention relates to the Primers suitable for amplification of iNOS gene region containing one or more polymorphic sites, said primers include:
(a) 5' CAG CGG AGT GAT GGC AAG CAC GAC 3' (SEQ ID No.2), which is a forward primer
(b) SEQ ID 3: 5' GAT GCA CAG CTG GGG AAC AAG ACG 3' (SEQ ID No.3), which is a reverse primer

The following examples are given by way of illustration of the present invention and should not be construed to limit the scope of the present invention.

### EXAMPLES

### EXAMPLE 1

### Identification of the marker gene:

Taking in consideration the important functions of NO at HA, iNOS, the inducible nitric oxide synthase was selected as the candidate gene for the study.

### EXAMPLE 2

### Selection of the study subjects:

Clinical severity of HAPE was assessed by Lake Louise acute mountain sickness (AMS) scoring system. Briefly, the patients were assessed for the presence of five symptoms: headache, gastrointestinal upset, fatigue, weakness, or both, dizziness, lightheadedness, or both, and difficulty in sleeping. Change in mental status, ataxia and peripheral edema were also assessed. Each of these symptoms were rated between 0 and 3. A score of 0 indicated no symptoms; 1, mild symptoms; 2, moderate symptoms; and 3, severe symptoms. HAPE score is the sum of all 8 symptoms and patients were characterized by HAPE score>6 (Anand IS et al 1998). LLs were subjects who even after induction to high altitudes atleast thrice never found to have any of the above mentioned symptoms. HA natives were the permanent residents of HA from ancient times.

### EXAMPLE 3

### Extraction of genomic DNA from leukocytes:

Genomic DNA was extracted from blood using salting out method. Lysis of red blood cells in presence of high salt was followed by treatment with Nucleus lysis buffer (NLB). Proteins were precipitated and DNA was extracted from peripheral blood leukocytes using a modification of the salting out procedure. The concentration of the DNA was determined by measuring the optical density of the sample, at a wavelength of 260 nm. (Miller SA et al 1988).

### EXAMPLE 4

### Identification of the allelic variants of the iNOS gene:

This example describes the identification of allelic variants of iNOS gene by PCR and sequencing using certain oligonucleotide primers according to the invention. The DNA was then amplified by polymerase chain reaction by using the oligonucleotide primers:
1. 5'CAG CGG AGT GAT GGC AAG CAC GAC 3'(as listed in SEQ ID NO:2) and
2. 5' GAT GCA CAG CTG GGG AAC AAG ACG 3'(as listed in SEQ ID NO:3). Polymerase chain reaction was carried out using the following conditions:
   Step 1 94°C for 4 min
   Step 2 94°C for 30 sec
   Step 3 62.5°C for 30 sec
   Step 4 72°C for 45 sec
   Step 5 34 times to Step 2
   Step 6 72°C for 10 min

PCR was performed in a Perkin Elmer GeneAmp PCR System 9600. This reaction produced a DNA fragment of 258bp when analyzed by 2% agarose gel electrophoresis. The PCR product was purified from band cut out of agarose gel using a Amersham Pharmacia gel extraction kit (Amersham) and both the strands of the PCR product were directly sequenced using dye terminator chemistry on an ABI Prism 377 automated DNA sequencer. The PCR product was identical to the human iNOS gene sequence except of the novel single base pair change mentioned in Table 1.

### EXAMPLE 5

### Nucleotide sequence of the Allelic Variant of the iNOS gene:

The nucleotide sequence of the allelic variant of iNOS gene derived using the method as described in example 1 -

5'CAG CGG AGT GAT GGC AAG CAC GAC TTC CGG GTG TGG AAT GCT CAG
CTC ATC CGC TAT GCT GGC TAC CAG ATG CCA GAT GGC AGC ATC AGA
GGG GAC CCT GCC AAC GTG GAA TTC ACT CAG GTA CCC GGC CCA GCC
TCA GCC A*/GCC GGC CAT TGG GGC GGG GAG CCC CGT GGT GAG CGA GTG
ACA GAG TGG AGC CCA GAG GAG ACA CGC AGC CCG GGC TTA CAG ACT
CAC AGG GCC CGT CTT GTT CCC CAG CTG TGC ATC 3'

In the above sequence the SNP* is shown in bold.

### EXAMPLE 6

G allele is related with adaptation and A allele associates with the disease:
A method as described in example 4 is applied to a series of DNA samples extracted from HA natives, HAPE controls and HAPE patients. A highly significant association of G allele with the HA adaptation and A allele with the disease has been observed. The results are summarized in the table below:

| Association type | χ² value | p value | Odds ratio | 95% CI | Relative risk |
|---|---|---|---|---|---|
| HAPE patients & HAPE controls | 10.63 | 0.001 | 2.56 | 1.45-4.54 | 1.66 (1.21-2.27) |
| HAPE patients & HA natives | 33.96 | <0.001 | 6.29 | 3.30-12.01 | 3.22 (2.05-5.06) |
| HAPE controls & HA natives | 7.42 | 0.006 | - | - | - |

Hence, individuals with GG genotype being at low risk and those with AA genotype being at high risk for HAPE, can be expected to hold true for other populations also.

### EXAMPLE 7

### Nucleic acid vectors containing the iNOS variant sequences:

Vectors and host cells transformed with the allelic variants of the iNCS gene containing one or more polymorphic sites as listed in table 1, can be prepared, for example, as detailed below.

Variant genes can be expressed in an expression vector in which a variant gene is operably linked to a native or other promoter. Usually, the promoter is eukaryotic promoter for expression in a mammalian cell. The transcription regulation sequences typically include a heterologous promoter and optionally an enhancer, which is recognized by the host. The selection of an appropriate promoter, for example trp, lac, phage, glycolytic enzyme and tRNA, depends on the host selected. Commercially available expression vectors can also be used. Suitable host cells include bacteria such as E.coli, yeast, filamentous fungi, insect cells, mammalian cells, typically immortalized, e.g., mouse, CHO, human and monkey cell lines and derivatives thereof. Preferred host cells are able to process the variant gene product to produce an appropriate mature polypeptide.

### Advantages of the present invention:

The present invention adds following points to the treatment of HAPE.
1. Inducible nitric oxide synthase gene as a novel marker for HAPE studies.
2. Novel primer sequences responsible for the amplification of PCR product containing novel SNP.
3. SNP (19480 A/G) that can be used for further association studies.
4. A significant association of wild type allele (A) to the disease (Table 2 and 3).
5. A significant association of mutant allele (G) to adaptation (Table 2 and 3).
6. A significant difference between the frequency of alleles with respect to HA native and HAPE controls (Table 2 and 3).
7. The presence of G allele predisposes an individual to less chances of getting diseased.
8. It may help individuals to decide visiting high altitude for various reasons.
**Provided below is the sequence listing information for SEQ ID Nos. 1, 2 and 3**

### SEQUENCE LISTING

### GENERAL INFORMATION

### APPLICANT: CSIR

TITLE OF INVENTION: Method for the detection of predisposition to high altitude pulmonary edema (HAPE).

### NUMBER OF SEQUENCES: 03

CORRESPONDING ADDRESS: Institute of genomics and integrative biology, CSIR, Delhi University Campus, Mall Road-110007, India.
Telephone: +91-11-27666156 Fax: +91-11-27667471

### INFORMATION FOR SEQUENCE ID NO: 1

### 1. SEQUENCE CHARACTERISTICS:

1. LENGTH: 258 bp
2. TYPE: DNA 
5'CAG CGG AGT GAT GGC AAG CAC GAC TTC CGG GTG TGG AAT GCT CAG
CTC ATC CGC TAT GCT GGC TAC CAG ATG CCA GAT GGC AGC ATC AGA
GGG GAC CCT GCC AAC GTG GAA TTC ACT CAG GTA CCC GGC CCA GCC
TCA GCC A*/GCC GGC CAT TGG GGC GGG GAG CCC CGT GGT GAG CGA GTG
ACA GAG TGG AGC CCA GAG GAG ACA CGC AGC CCG GGC TTA CAG ACT
CAC AGG GCC CGT CTT GTT CCC CAG CTG TGC ATC 3'
3. ORGANISM: *Homo sapiens* (Humans)
4. IMMEDIATE SOURCE: PCR
5. NAME/KEY: Marker Region
6. SEQUENCE ID # 1

### INFORMATION FOR SEQUENCE ID NO: 2

### 1. SEQUENCE CHARACTERISTICS:

LENGTH: 24 bp

TYPE: DNA
5'CAG CGG AGT GAT GGC AAG CAC GAC 3'

ORGANISM: Artificial sequence

IMMEDIATE SOURCE: Synthetic

NAME/KEY: Synthetic Oligonucleotide

SEQUENCE ID # 2

### INFORMATION FOR SEQUENCE ID NO: 3

### 1. SEQUENCE CHARACTERISTICS

LENGTH: 24 bp

TYPE: DNA
5' GAT GCA CAG CTG GGG AAC AAG ACG 3'

ORGANISM: Artificial sequence

IMMEDIATE SOURCE: Synthetic

NAME/KEY: Synthetic Oligonucleotide

SEQUENCE ID # 3

### References

1. Houston CS. Acute pulmonary edema of high altitude. N Engl J Med 1960;263:478-480.
2. Bartsch P. High altitude pulmonary edema. Respiration 1997;64:435-443.
3. Bartsch P, Vock P et al Respiratory symptoms, radiographic and physiologic correlations at high altitude. Hypoxia: the adaptations. 1990;241-245.
4. Hackett PH, Roach RC. High altitude medicine. J Wilderness Med 1990;1:3-26.
5. Lobenhoffer HP, Zink RA et al. High altitude pulmonary edema: analysis of 166 Cases: High altitude physiology and medicine New York, NY: Springer-Verlag; 1982:219-231.
6. Scherrer U, Vollenweider L et al. Inhaled nitric oxide for high altitude pulmonary edema. N Engl J Med 1996;334:624-629.
7. Bartsch P, Maggiorini Metal. Prevalence of high altitude pulmonary edema by nifedipine. N Engl J Med 1991;325:1284-1289.
8. Palmer RMJ, Ferrige AG, Moncada S (1987) Nitric Oxide release accounts for the biological activity of endothelium derived relaxing factor. Nature 327:524-526.
9. Tomas C. Bellamy, John Wood et al. On the activation of soluble guanylyl cyclase by nitric oxide. PNAS 2002 99: 507-510.
10. Anand IS, Prasad BAK et al. Effects of inhaled NO and O2 in high altitude pulmonary edema. Circulation 1998; 98:2441-2445.
11. Goldstein I, Lue TF. Oral sildenafil in the treatment of erectile dysfunction. N Engl J Med 1998; 338:1397-1404.
12. Busch T, Bertsch P et al. Hypoxia decrease exhaled nitric oxide in mountaineers susceptible to high altitude pulmonary edema. Am J Respir Crit Care Med 2001; 163:368-373.
13. Michel T, Feron O. Nitric oxide synthases: which, where, how, and why? J Clin Invest. 1997; 100:2146-2152.
14. Xia Y, Roman LJ et al. Inducible nitric-oxide synthase generates superoxide from the reductase domain. J Biol Chem.1998; 273:22635-22639.
15. MA Qadar Pasha, AP khan, Ratan Kumar, SK Grover, RB Ram, T Norboo, KK Srivastava, W Selvamurthy, SK Brahmachari. Angiotensin Converting enzyme insertion allele in relation to high altitude adaptation. Ann Hum Genet 2001; 65 (6): 531-536.
16. Hackett PH, Roach RC et al. High altitude illness. N Engl J Med 2001;345(2):107-113.
17. Droma Y, Hanaoka M et al. Positive Association of the Endothelial Nitric Oxide Synthase Gene Polymorphisms With High-Altitude Pulmonary Edema.Circulation 2002;106: 826-830.
18. Monti LD, Barlassina C. Endothelial nitric oxide synthase polymorphisms are associated With Type 2 Diabetes and the Insulin resistance syndrome. Diabetes. 2003;52(5):1270-1275.
19. Via M, Lopez-Alomar A. Lack of association between eNOS gene polymorphisms and ischemic heart disease in the Spanish population. Am J Med Genet 2003;30:116A(3):243-8.
20. Miller, S.A., Dykes, D.D et al. A simple salting out procedure for extracting DNA from human nucleated cells. Nucleic Acids Research 1988; 16: 1215.

## Claims

1. A method for detecting predisposition to high altitude pulmonary edema (HAPE) in a subject, said method comprising identifying a polymorphism in an inducible nitric oxide synthase gene of said subject, wherein said polymorphism is at position 142 of SEQ ID NO: 1, wherein predisposition to HAPE is indicated by the presence of the nucleotide A at said position.

2. A method of claim 1 wherein the subject is human.

3. An isolated polynucleotide for detecting a predisposition to high altitude pulmonary edema (HAPE) in a human subject, wherein said polynucleotide is selected from the group consisting of a forward primer comprising the sequence set forth in SEQ ID NO: 2 and a reverse primer comprising the sequence set forth in SEQ ID NO: 3.

4. A kit for detecting a predisposition to HAPE in a human subject, said kit comprising a pair of primers suitable for amplification of iNOS gene region containing one or more polymorphic sites, said primers include
(a) 5' CAG CGG AGT GAT GGC AAG CAC GAC 3' (SEQ ID NO: 2), which is a forward primer
(b) 5' GAT GCA CAG CTG GGG AAC AAG ACG 3' (SEQ ID NO: 3), which is a reverse primer.

5. The use of the polymorphism in an inducible nitric oxide synthase that is at position 142 of SEQ ID NO: 1 for detecting predisposition to high altitude pulmonary edema (HAPE).

## Patentansprüche

1. Verfahren zum Nachweisen einer Prädisposition für höhenbedingtes Lungenödem (HAPE) bei einem Subjekt, wobei das Verfahren das Identifizieren eines Polymorphismus in einem induzierbaren Stickoxidsynthase-Gen des Subjekts umfasst, wobei sich der Polymorphismus in Position 142 von SEQ ID NO: 1 befindet, wobei die Prädisposition für HAPE durch die Anwesenheit des Nucleotids A in dieser Position angezeigt wird.

2. Verfahren nach Anspruch 1, wobei es sich um ein humanes Subjekt handelt.

3. Isoliertes Polynucleotid zum Nachweisen einer Prädisposition für höhenbedingtes Lungenödem (HAPE) bei einem humanen Subjekt, wobei das Polynucleotid aus der Gruppe ausgewählt ist, die aus einem Vorwärtsprimer, der die in SEQ ID NO: 2 angegebene Sequenz umfasst, und einem Rückwärtsprimer, der die in SEQ ID NO: 3 angegebene Sequenz umfasst, besteht.

4. Kit zum Nachweisen einer Prädisposition für HAPE bei einem humanen Subjekt, wobei das Kit ein Paar von Primern umfasst, die zur Amplifikation der iNOS-Genregion mit einem Gehalt an einer oder mehreren polymorphen Stellen geeignet sind, wobei die Primer
(a) 5' CAG CGG AGT GAT GGC AAG CAC GAC 3' (SEQ ID NO: 2), das einen Vorwärtsprimer darstellt,
(b) 5' GAT GCA CAG CTG GGG AAC AAG ACG 3' (SEQ ID NO: 3), das einen Rückwärtsprimer darstellt,
umfassen.

5. Verwendung des Polymorphismus in einer induzierbaren Stickoxidsynthase, der sich in Position 142 von SEQ ID NO: 1 befindet, zum Nachweisen einer Prädisposition für höhenbedingtes Lungenödem (HAPE).

## Revendications

1. Procédé de détection de la prédisposition à l'oedème pulmonaire de haute altitude (HAPE) chez un sujet, ledit procédé comprenant l'identification d'un polymorphisme dans un gène de l'oxyde nitrique-synthase inductible (NOSi) dudit sujet, où ledit polymorphisme est à la position 142 de SEQ ID NO:1, où la prédisposition à l'HAPE est indiquée par la présence du nucléotide A à ladite position.

2. Procédé selon la revendication 1, où le sujet est humain.

3. Polynucléotide isolé destiné à détecter une prédisposition à l'oedème pulmonaire de haute altitude (HAPE) chez un sujet humain, où ledit polynucléotide est sélectionné dans le groupe constitué par une amorce sens comprenant la séquence indiquée dans SEQ ID NO: 2 et une amorce anti-sens comprenant la séquence indiquée dans SEQ ID NO:3.

4. Kit de détection d'une prédisposition à l'HAPE chez un sujet humain, ledit kit comprenant une paire d'amorces convenant à l'amplification de la région du gène de NOSi contenant un ou plusieurs sites polymorphiques, lesdites amorces comprenant
(a) 5' CAG CGG AGT GAT GGC AAG CAC GAC 3' (SEQ ID NO: 2), qui est une amorce sens
(b) 5' GAT GCA CAG CTG GGG AAC AAG ACG 3' (SEQ ID NO : 3), qui est une amorce anti-sens.

5. Utilisation du polymorphisme dans une oxyde nitrique-synthase inductible qui est à la position 142 de SEQ ID NO: 1 pour détecter la prédisposition à l'oedème pulmonaire de haute altitude (HAPE).
